# FASCICULE DE BREVET EUROPEEN

(11) **EP 2 496 708 B1**
(45) Date de publication et mention de la délivrance du brevet: **01.03.2017**
(21) Numéro de dépôt: 10781460.0
(22) Date de dépôt: 05.11.2010
(51) Int. Cl.: C12P 17/18, C07D 493/22, A61K 31/365, A61P 17/04, A61P 17/06

(54) **PROCEDE DE PRODUCTION DE TRIPTOLIDE**
VERFAHREN ZUR HERSTELLUNG VON TRIPTOLID
METHOD FOR PRODUCING TRIPTOLIDE

(30) Priorité: 05.11.2009 FR 0957847
(43) Date de publication de la demande: 12.09.2012
(73) Titulaire: Pierre Fabre Médicament, 92100 Boulogne-Billancourt (FR)
(72) Inventeur: STEWARD, Nicolas, F-31860 Pins-justaret (FR); CHOMARAT, Nadine, F-81150 Fayssac (FR); N'GUYEN, Ngoc Thien, F-31180 Rouffiac Tolosan (FR)
(74) Mandataire: Regimbeau
(86) Numéro de dépôt international: PCT/EP2010/066916
(87) Numéro de publication internationale: WO 2011/054929

(56) Documents cités:
- WO-A1-97/44476
- WO-A1-98/13057
- WO-A1-2005/012507
- JP-A- 57 002 699
- US-A- 4 328 309
- KUTNEY JAMES P ET AL: "Cytotoxic diterpenes triptolide, tripdiolide, and cytotoxic triterpenes from tissue cultures of Tripterygium wilfordii", CANADIAN JOURNAL OF CHEMISTRY, NRC RESEARCH PRESS, CA, vol. 59, no. 17, 1 janvier 1981 (1981-01-01), pages 2677-2683, XP002592559, ISSN: 0008-4042, DOI: 10.1139/V81-385 cité dans la demande
- KUTNEY, J.P., HAN, K.: "Studies with plant-cell cultures of the Chinese herbal plant, Tripterygium wilfordii. Isolation and characterization of diterpenes", RECUEIL DES TRAVAUX CHIMIQUES DES PAYS-BAS / JOURNAL OF THE ROYAL NETHERLANDS, vol. 115, no. 1, 1996, pages 77-93, XP8124425,
- Takayama S.: "Tripterygium wilfordii: In vitro culture and the production of the anticancer compounds tripdiolide and triptolide (Chapter XXVIII)"; "Medicinal and aromatic plants VII" In: Bajaj Y.P.S. (ed.): "Biotechnology in argriculture and forestry", 1994, Springer-Verlag, Berlin, Germany, XP8124468, vol. 28, pages 457-468, pages 458-466, alinéas 2.1-2.4, 3.; figures 3-7; tableaux 1-3
- Samija, MD: "Enhanced yield of medicinal products from Tripterygium cell cultures (Thesis)", The University of British Columbia, UBC library, Vancouver, Canada, 1 février 1992 (1992-02-01), pages I-iv, 26-51,67-88,116,137-139, XP002592561, Extrait de l'Internet: URL:http://circle.ubc.ca/bitstream/handle/ 2429/2265/ubc_1992_spring_samija_mijo.pdf? sequence=1 [extrait le 2010-07-19]
- KUTNEY J P ET AL: "Cultivation of Tripterygium wilfordii Tissue Cultures for the Production of the Cytotoxic Diterpene Tripdiolide.", PLANTA MEDICA, vol. 48, no. 7, juillet 1983 (1983-07), pages 158-163, XP8124443, ISSN: 0032-0943
- KUTNEY J P ET AL: "Studies with Plant Cell Cultures of the Chinese Herbal Plant, Tripterygium wilfordii. Synthesis and Biotransformation of Diterpene Analogues", HETEROCYCLES, ELSEVIER SCIENCE PUBLISHERS B.V. AMSTERDAM, NL, vol. 44, no. 1, 1 janvier 1997 (1997-01-01), pages 95-104, XP008124404, ISSN: 0385-5414, DOI: 10.3987/COM-96-S25
- DATABASE WPI Week 200914 Thomson Scientific, London, GB; AN 2009-B11376 XP002592589, & CN 101 248 757 A (UNIV NON ENVIRONMENTAL POLLUTION PESTICIDE RES SERVICE CENT) 27 août 2008 (2008-08-27)
- DATABASE WPI Week 200415 Thomson Scientific, London, GB; AN 2004-144403 XP002592590, & CN 1 434 123 A (BEIJING DAKEHAO SCI & TECHNOLOGY CO LTD) 6 août 2003 (2003-08-06)
- DATABASE WPI Week 198308 Thomson Scientific, London, GB; AN 1983-18319K XP002592591, & JP 58 005196 A (KYOWA HAKKO KOGYO KK) 12 janvier 1983 (1983-01-12)
- DUJACK L W ET AL: "ANTI NEOPLASTIC DI TERPENES IN TRIPTERYGIUM-WILFORDII TISSUE CULTURE THE EFFECTS OF PRECURSORS ON DI TERPENE PRODUCTION", IN VITRO; 31ST ANNUAL MEETING OF THE TISSUE CULTURE ASSOCIATION, ST. LOUIS, MO., USA, JUNE 1-5, 1980, TISSUE CULTURE ASSOCIATION, US, vol. 16, no. 3, 1 mars 1980 (1980-03-01), page 216, XP008124540, ISSN: 0073-5655, DOI: 10.1007/BF02616149 [extrait le 2007-05-01]
- KUTNEY J P ET AL: "Studies with tissue cultures of the Chinese herbal plant, Tripterygium wilfordii. Isolation of metabolites of interest in rheumatoid arthritis, immunosuppression, and male contraceptive activity", CANADIAN JOURNAL OF CHEMISTRY, vol. 70, no. 5, 1992, pages 1455-1480, XP002650436, ISSN: 0008-4042
- Dharmananda S: "TRIPTERYGIUM", Institute for Traditional Medicine, Portland, Oregon, USA, avril 1996 (1996-04), XP002650437, Extrait de l'Internet: URL:http://www.itmonline.org/arts/triptery .htm [extrait le 2011-07-14]
- TAO XUELIAN ET AL: "Effective therapy for nephritis in (NZB x NZW)F1 mice with triptolide and tripdiolide, the principal active components of the Chinese herbal remedy Tripterygium wilfordii Hook F", ARTHRITIS & RHEUMATISM, JOHN WILEY & SONS, INC, US, vol. 58, no. 6, 1 juin 2008 (2008-06-01), pages 1774-1783, XP002591440, ISSN: 0004-3591, DOI: 10.1002/ART.23513 [extrait le 2008-05-31]
- XU W Y ET AL: "Tripterygium in dermatologic therapy", INTERNATIONAL JOURNAL OF DERMATOLOGY, vol. 24, no. 3, avril 1985 (1985-04), pages 152-157, XP002650438, ISSN: 0011-9059
- TAO X ET AL: "The chinese anti-inflammatory and immunosuppressive herbal remedy Tripterygium wilfordii Hook F", RHEUMATIC DISEASE CLINICS OF NORTH AMERICA, vol. 26, no. 1, 1 février 2000 (2000-02-01), pages 29-50, XP000961355, ISSN: 0889-857X, DOI: 10.1016/S0889-857X(05)70118-6
- BEIER R C ET AL: "Occurrence of the toxin dehydroabietic acid in Salmonella typhimurium.", TOXICON : OFFICIAL JOURNAL OF THE INTERNATIONAL SOCIETY ON TOXINOLOGY MAR 2000, vol. 38, no. 3, March 2000 (2000-03), pages 337-346, ISSN: 0041-0101

## Description

L'invention concerne un procédé de production de triptolide à partir d'une culture de cellules en suspension de *Tripterygium sp.,* par exemple *Tripterygium wilfordii.*

Le Triptolide, un triépoxide diterpénique, est un composé purifié à partir de *Tripterygium wilfordii.* Cette plante a été utilisée depuis plus de 4 siècles dans la médecine traditionnelle chinoise dans le traitement des maladies auto-immunes, des maladies inflammatoires et en particulier dans la polyarthrite rhumatoïde. Récemment, on a découvert également la puissante activité anti-cancéreuse du Triptolide. Les activités anti-prolifératives et pro-apototiques ont été démontrées dans différents types de cellules cancéreuses *in vitro* et *in vivo.* Des essais cliniques ont été menés dans le traitement de la polyarthrite rhumatoïde et chez le patient atteint de cancer, par exemple d'une leucémie du sang, à stade avancé. Une publication récente (Brinker AM et col, Phytochemistry 68 (2007) 732-766) résume les propriétés pharmacologiques du triptolide et de ses dérivés issus de *Tripterygium wilfordii.*

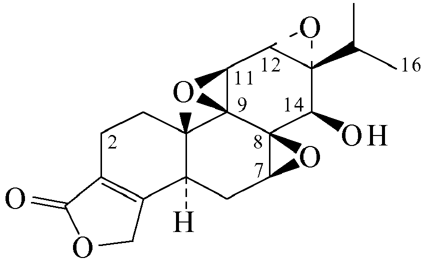

### Structure chimique du Triptolide

Le Triptolide est un métabolite secondaire appartenant à la famille des diterpènes qui sont naturellement présents en très faibles quantités dans les écorces et dans les parties aériennes de la plante, mais davantage dans les racines (teneur moyenne = 10 ppm).

La synthèse chimique du Triptolide est très difficile car elle nécessite la mise en oeuvre d'un procédé contenant environ 20 étapes.

Actuellement, le triptolide est fourni par la société PHARMAGENESIS. Il est produit par extraction de racines de *Tripterigium wilfordii* et purification par 2 étapes chromatographiques. Ce procédé est complexe et long.

Le rendement d'extraction/purification, par exemple, à partir des racines est de 0,0005%.

Il faut 10 à 15 ans pour que la plante arrive à maturité avant arrachage ; la production de triptolides à partir de la plante mature entraine la destruction de celle-ci. Dans un contexte écologique de développement durable, il est donc nécessaire de fournir une méthode alternative pour la production de Triptolide, et permettant un rendement applicable à la production industrielle du composé d'intérêt.

Kutney JP *et al.* divulguent un procédé de culture de cellules de feuilles de *Tripterygium wilfordii* en suspension et un procédé de séparation du triptolide et de ses dérivés à partir de cette culture (Can J Chem 58 (1981):2677-2683). Cependant, les rendements de production sont encore plus faibles que ceux du procédé classique.

La demande CN101358180A, publiée le 04 fév 2009, décrit un procédé de culture en suspension de cellules souches. Ces cellules ont été générées à partir des racines de *Tripterygium wilfordii.* La productivité en Triptolide annoncée atteint 0,027 mg/L par jour. Les auteurs de ce brevet décrivent, inter alia, un procédé constitué d'un milieu de propagation cellulaire, d'un milieu de production de triptolide et un milieu de production d'alcaloïdes totaux. Cependant, il n'est pas employé d'éliciteur abiotique tel que décrit selon la présente invention de précurseurs de la biosynthèse des terpènes, ni d'étape de sevrage hormonal.

La présente invention fournit un procédé de production de triptolide à partir de cellules souches de parties aériennes de *Tripterygium wilfordii* présentant un rendement élevé approprié à la production industrielle.

Ils sont ici divulgués les milieux de culture qui ont permis d'atteindre ce rendement particulièrement intéressant.

En effet, les inventeurs ont observé, de manière surprenante, un rendement en Triptolide de 3 mg/L de culture et par jour. Ce rendement est 110 fois supérieur au meilleur rendement global décrit dans la demande CN101358180A ; 73 fois supérieur au meilleur rendement dans le surnageant et 127 fois supérieur au meilleur rendement en pourcentage massique de triptolide par rapport au pourcentage massique de la biomasse sèche.

De manière surprenante, les inventeurs ont également montré qu'un extrait enrichi en triptolide obtenu par ce procédé inhibe l'activation du facteur de transcription NF-κB par le TNFα, ce de façon équivalente au triptolide pur.

Ils ont également montré qu'un extrait enrichi en triptolide obtenu selon le procédé de l'invention inhibe la production de N02- induite par les lipopolysaccharides, ce plus efficacement que le triptolide pur.

Ils ont également montré qu'un extrait enrichi en triptolide obtenu selon le procédé de l'invention inhibe l'influx de calcium intracellulaire induit suite à la stimulation spécifique des récepteurs PAR-2 par la trypsine.

Ces trois tests valident l'activité anti-inflammatoire d'un extrait enrichi en triptolide obtenu selon le procédé de l'invention.

Cette activité est confirmée au niveau cutané dans les modèles dermatite atopique et psoriasis par puces à ARN (PCR array).

L'invention concerne par conséquent un procédé de production de Triptolide à partir d'une culture de cellules en suspension de parties aériennes de *Tripterygium sp.,* par exemple de *Tripterygium wilfordii*, *Tripterygium regeli*, ou *Tripterygium hypoglaucum.* Dans un mode de réalisation de l'invention, le procédé de production de Triptolide est réalisé à partir d'une culture de cellules en suspension de parties aériennes, par exemple les tiges, pétioles, feuilles, et/ou inflorescences.

La présente invention concerne un procédé de production de triptolide dans le milieu de culture à partir d'une culture cellulaire de l'espèce *Tripterygium* comprenant les étapes suivantes :
(i) une phase de production de biomasse de cellules dédifférenciées de l'espèce *Tripterygium* dérivées de cals dans un ou des milieux nutritifs de propagation dans des conditions de multiplication de la biomasse,

- (ii) une phase de sevrage hormonal des cultures cellulaires obtenues à l'étape (i) dans un milieu de sevrage dans lequel l'acide 2,4 dichlorophénoxyacétique (2,4D) et l'acide naphthalène acétique (NAA) sont présents respectivement à une concentration inférieure à 0,01 mg/L de milieu de culture,
- (iii) une phase d'élicitation par ajoût d'un cocktail d'élicitation aux cellules de l'étape (ii) dans le milieu de sevrage,
   le cocktail d'élicitation comprenant:
   a) au moins un facteur de différenciation cellulaire des cellules végétales,
   b) au moins un agent stressant, et
   c) au moins un précurseur de la voie de synthèse des terpènes,
- (iv) préparation d'un extrait enrichi en triptolide à partir du milieu de culture à l'issue de l'étape (iii).

La phase d'élicitation réside dans l'ajout du cocktail d'élicitation dans du milieu de sevrage contenant la biomasse sevrée produite à l'étape (ii) et la mise en culture ; la production de triptolide a lieu dans ledit milieu de culture. La phase d'élicitation au sens de la présente invention correspond donc à la phase de production des triptolides.

Selon la présente invention le cocktail d'élicitation utilisé dans le procédé comprend :
a) au moins un facteur de différenciation cellulaire des cellules végétales, par exemple une cytokinine, par exemple choisie parmi la benzyl-aminopurine (BAP), l'acide abscissique, la kinétine, le thidiazuron, la 6-γ-γ-dimethylallylaminopurine, la zéatine, l'isopentényladénine, ou une gibbérelline;
b) au moins un agent stressant, par exemple un éliciteur abiotique et
c) au moins un précurseur de la voie de synthèse des terpènes, par exemple de la synthèse de triptolide, par exemple le géraniol, le farnésol, y compris leurs formes pyrophosphates, l'acétate de sodium, l'acide pyruvique, ou l'acide mévalonique.

Préférentiellement, l'étape (i) du procédé de l'invention est précédée des étapes suivantes :
(α) Induction de cals à partir d'un explant de tissu de partie aérienne de *Tripterygium wilfordii* par mise en culture sur un milieu gélosé comprenant des inducteurs de dédifférenciation cellulaire
(β) Mise en suspension de cellules de cals obtenus à l'étape (i) et propagation des cellules de la suspension en milieu de propagation.

On entend par « parties aériennes » les parties de la plante situées au-dessus du sol, par exemple, les feuilles, les tiges, les pétioles et/ou les inflorescences.

Ledit procédé peut également être appliqué à toute autre partie de la plante comme les graines et les racines.

L'étape (α) du procédé consiste en la production de cals à partir d'un explant de tissu, par exemple d'un explant de partie(s) aérienne(s) de *Tripterygium wilfordii*, par exemple un morceau de feuille d'une taille de 1 cm² environ, mise en culture sur un milieu gélosé comprenant des inducteurs de dédifférenciation.

Selon un mode de réalisation la ou les partie(s) aérienne(s) de *Tripterygium wilfordii* sont des feuilles, tiges, pétioles et/ou des inflorescences.

On entend par « cal » un amas de cellules dédifférenciées également appelées cellules souches.

Le milieu de dédifférenciation est par exemple un milieu comprenant :
- au moins un macroélément, par exemple choisi parmi le NH₄NO₃, le KNO₃, CaCl₂.2H₂O, MgSO₄.7H₂O, KH₂PO₄, par exemple à une concentration pouvant aller jusqu'à 6000 mg/L,
- au moins un microélément, par exemple KI, H₃BO₃, MnSO₄.4H₂O, ZnSO₄.H₂O, Na₂MoO₄.2H₂O, CuSO₄.5H₂O, CoCl₂.6H₂O, FeSO₄.7H₂O, Na₂EDTA.2H₂O, par exemple à une concentration de microélément totale dans ledit milieu pouvant aller jusqu'à 200 mg/L de milieu culture,
- au moins une vitamine, par exemple le myo-Inositol, l'acide Nicotinique, Pyridoxine-HCl, Thiamine-HCl, par exemple à une concentration pouvant aller jusqu'à 3 g/L de milieu de culture,
- au moins un acide aminé par exemple à une concentration dans le milieu de culture pouvant aller jusqu'à 3 g/L, par exemple de la glycine,
- au moins une source de carbone, par exemple du saccharose, par exemple à une concentration de 20 à 70 g/L de milieu de culture par exemple à 30 g/L,
- au moins une hormone de préférence végétale ou facteur de croissance de préférence végétal ou régulateur de croissance de préférence végétal, par exemple, la kinétine, l'acide 2,4 dichlorophénoxyacétique (2,4D), l'acide Naphthalène acétique (NAA), par exemple à une concentration de 0,001 à 10 mg/L de milieu de culture , par exemple de 0,1 à 3 mg/L de milieu de culture.

Des compositions de milieu de dédifférenciation et leur utilisation sont données dans les exemples.

Le pH dudit milieu est ajusté, par exemple à pH 6 ± 0,5, et autoclavé à 121°C pendant un minimum de 20 minutes ou par filtration sur 0,2µm.

L'incubation peut avoir lieu à l'obscurité, par exemple à une température d'environ 25-30°C, par exemple à 27°C ou 28°C.

Le milieu de dédifférenciation est, par exemple un milieu solide, par exemple gélifié par ajout de 8 à 12 g/L d'agar, par exemple 8g/L.

L'étape (β) du procédé consiste en la mise en suspension de cellules dédifférenciées issues de cals obtenus par la première étape dans un milieu de culture liquide et la propagation des cellules de la suspension. La culture a lieu pendant une période de 10 à 30 jours, par exemple de 15 à 20 jours, par exemple à une température d'environ 27°C. La culture a lieu à l'obscurité et sous agitation.

Le milieu de culture de cette étape (β) est, par exemple, le milieu propagation cellulaire, par exemple ajusté à pH 6 et stérilisé par autoclavage à 121°C pendant un minimum de 20 minutes ou par filtration stérilisante sur filtre 0,2µm :
Le milieu de propagation cellulaire est un milieu comprenant :
   - au moins un macroélément, par exemple choisi parmi le NH₄NO₃, le KNO₃, CaCl₂.2H₂O, MgSO₄.7H₂O, KH₂PO₄, par exemple à une concentration pouvant aller jusqu'à 6000 mg/L,
   - au moins un microélément, par exemple KI, H₃BO₃, MnSO₄.4H₂O, ZnSO₄.H₂O, Na₂MoO₄.2H₂O, CuSO₄.5H₂O, CoCl₂.6H₂O, FeSO₄.7H₂O, Na₂EDTA.2H₂O, par exemple à une concentration de microélément totale dans ledit milieu pouvant aller jusqu'à 200 mg/L de milieu culture,
   - au moins une vitamine, par exemple le myo-Inositol, l'acide Nicotinique, Pyridoxine-HCl, Thiamine-HCl, par exemple à une concentration pouvant aller jusqu'à 3 g/L de milieu de culture,
   - au moins un acide aminé par exemple à une concentration dans le milieu de culture pouvant aller jusqu'à 3g/L, par exemple de la glycine,
   - au moins une source de carbone, par exemple du saccharose, par exemple à une concentration de 30g/L,
   - au moins une hormone de préférence végétale ou facteur de croissance de préférence végétal ou régulateur de croissance de préférence végétal, par exemple, la kinétine, l'acide 2,4 dichlorophénoxyacétique (2,4D), l'Acide Naphthalène acétique (NAA), par exemple à une concentration de 0,001 à 10 mg/L, par exemple de 0.1 à 3 mg/L de milieu de culture.

Un exemple de milieu de propagation et son utilisation est fourni dans les exemples.

Un milieu de propagation est par exemple le milieu de l'exemple 2.

Selon une alternative, les étapes de dédifférenciation (α) et/ou de propagation (β) peuvent être réalisées toutes les deux dans un milieu de propagation ou un milieu de dédifférenciation.

L'étape (i) du procédé de l'invention consiste en la production de biomasse à partir de cellules dédifférenciées de l'espèce *Tripterygium* dérivées de cals, par exemple des cellules de la suspension obtenue à l'étape (β) dans un milieu nutritif approprié, par exemple le milieu de propagation décrit ci-dessus. Elle dure de 10 à 30 jours. Elle se déroule de préférence à 27-28°C.

Au cours de cette étape, les cellules sont régulièrement « repiquées » ou propagées, par exemple tous les 7 à 10 jours. Le repiquage consiste à diluer une partie de la culture cellulaire dans du milieu neuf. Par exemple, 1/5^{eme} de la culture est remise en suspension dans un volume de milieu neuf correspondant au volume de la culture initiale Il permet l'entretien de la lignée cellulaire en milieu liquide.

De la même façon, on peut procéder à un accroissement de la quantité de biomasse en utilisant une culture entière pour inoculer un milieu nutritif neuf, l'inoculum représentant environ 1/5^{ème} du volume final de culture.

L'étape (ii) du procédé de l'invention consiste en une étape de sevrage hormonal, par exemple d'une durée de 5 à 15 jours, par exemple d'environ 7 jours.

Le sevrage hormonal a pour objectif d'éliminer les auxines, comme l'hormone de croissance 2,4-D et/ou la NAA, présentes dans le milieu de culture ou de propagation. Cette étape permet d'obtenir une synchronisation métabolique des cellules, c'est-à-dire, la dérépression de la voie de biosynthèse des terpènes.

Le milieu de sevrage hormonal est un milieu ne contenant pas d'auxines, par exemple ne contenant pas de 2,4D, ni de NAA, ou un milieu substantiellement exempt d'auxine, c.-à-d. un milieu dans lequel le 2,4D et le NAA sont présents respectivement à une concentration inférieure à 0,01 mg/L de milieu de culture.

Le milieu de sevrage a la composition suivante :
- au moins un macroélément, par exemple choisi parmi le NH₄NO₃, le KNO₃, CaCl₂.2H₂O, MgSO₄.7H₂O, KH₂PO₄, et pyruvate de sodim, par exemple à une concentration pouvant aller jusqu'à 7000 mg/L,
- au moins un microélément, par exemple KI, H₃BO₃, MnSO₄.4H₂O, ZnSO₄.H₂O, Na₂MoO₄.2H₂O, CuSO₄.5H₂O, CoCl₂.6H₂O, FeSO₄.7H₂O, Na₂EDTA.2H₂O, par exemple à une concentration de microélément totale dans ledit milieu pouvant aller jusqu'à 200 mg/L de milieu culture,
- au moins une vitamine, par exemple le myo-Inositol, l'acide Nicotinique, Pyridoxine-HCl, Thiamine-HCl, Glycine, par exemple à une concentration pouvant aller jusqu'à 60 mg/L de milieu de culture,
- au moins une source de carbone, par exemple du saccharose, par exemple à une concentration de 20 à 70 g/L, par exemple à 30g/L,
- au moins une hormone de préférence végétale ou facteur de croissance de préférence végétal ou régulateur de croissance de préférence végétal, par exemple, la kinétine, l'acide Indole-butyrique (IBA), par exemple à une concentration de 0,001 à 10 mg/L de milieu de culture, par exemple de 1 à 3 mg/L de milieu de culture.

Un milieu de sevrage selon l'invention est par exemple le milieu sevrage dont la composition est indiquée dans l'exemple 3. Le pH du milieu est ajusté, par exemple à pH 6 ± 0,5 et stérilisé par un moyen approprié.

La phase d'élicitation de l'étape (iii) du procédé de l'invention permet d'induire la production de triptolide à partir de la culture cellulaire sevrée. La phase d'élicitation est aussi la phase de production de triptolide. Elle dure de 15 à 35 jours, par exemple de 20 à 25 jours.

Les inventeurs ont en effet observé que la division cellulaire et la production de triptolide ne sont pas concomitantes. De manière surprenante, elles sont même incompatibles. Pour résoudre ce problème, les inventeurs ont mis au point une étape de sevrage hormonal et un cocktail d'agents éliciteurs qui stoppe la division cellulaire, induit un stress cellulaire qui active des voies de défense biochimiques provoquant la production de triptolide et fournit des précurseurs de la voie de biosynthèse des terpènes.

Selon la présente invention, ce cocktail ne contient pas d'auxines, par exemple il ne contient pas de 2,4D, ou pas de NAA, ou aucun des deux produits.

Selon l'invention, ce cocktail comprend :
1) au moins un facteur de différentiation cellulaire des cellules végétales, par exemple, une cytokinine, ou une gibbérilline, par exemple la benzyl-aminopurine (BAP),
2) au moins un agent stressant ou "éliciteur" , par exemple d'origine chimique ou "abiotiques", par exemple l'acide 5-chloro salicylique (5-Chloro SA), l'acide salicylique, l'acide acétylsalicylique (ASA), et/ou le méthyl-jasmonate (MeJA),
3) au moins un précurseur entrant dans la synthèse des terpènes comme par exemple le Farnesol, le Géraniol, l'acétate de sodium, l'acide pyruvique ou l'acide mévalonique.

Une cytokinine au sens de la présente invention est par exemple l'acide absicissique, la benzyl-amino purine, la zéatine, la kinétine, le thidiazuron, l'isopentényladénine, la 6-γ-γ-dimethylallylaminopurine, une gibbérelline.

La BAP est par exemple utilisée à une concentration comprise entre 0,01 et 5 mg/L, par exemple entre 0,5 et 5 mg/L de milieu de culture.

L'acide 5-chloro salicylique (5-Chloro SA) est par exemple utilisé à une concentration comprise entre 0,1 et 15 mg/L de milieu de culture.

L'acide salicylique est par exemple utilisé à une concentration comprise entre 0,1 et 100 mg/L de milieu de culture par exemple entre 20 et 60 mg/L, par exemple 45 mg/L de milieu de culture.

Le farnesol est présent à une concentration de 1 et 100 mg/L, par exemple de 15 à 30 mg/L, par exemple à 30 mg/L de milieu de culture.

Le géraniol est présent à une concentration de 1 et 100 mg/L, par exemple 20 à 30 mg/L de milieu de culture.

Le méthyl-jasmonate (MeJA) est présent à une concentration de 1 et 100 mg/L.

Ce cocktail éliciteur a une triple action : il réoriente les cellules vers une différenciation cellulaire, par exemple les racines ; il engendre un stress cellulaire activant des gènes impliqués dans la production de produits de réaction de défense chimique, par exemple les triptolides et/ou leurs dérivés, et apporte aux cellules de plantes des précurseurs de la synthèse des terpènes.

La composition du cocktail d'élicitation est par exemple la suivante : Benzyl-aminopurine (BAP) de 0,5 à 5 mg/L, par exemple 0,5 à 3 mg/L, par exemple 0,7 à 3mg/L, Acide 5-Chloro Salicylique (5-Chloro SA) de 2 à 6 mg/L, par exemple de 3 à 5 mg/L par exemple à 3 ou à 5 mg/L, Acide Acetyl-Salicylique (ASA) et/ou acide salycilique de 20 à 60 mg/L, par exemple 30 à 50 mg/L, par exemple, 33 ou 45 mg/L, Methyl-Jasmonate (MeJA) à 22,4 mg/L, Farnesol (F-OH) de 19 à 40 mg/L et Géraniol à 20 à 30 mg/L, la quantité en mg/L correspondant à des mg/L de milieu de culture. Il ne s'agit pas des concentrations des différents produits dans une solution mère.

Le cocktail d'élicitation est introduit, par exemple à l'aide de solutions mères concentrées réalisées dans du diméthyl sulfoxyde, dans le milieu de culture.

La phase d'élicitation (iii) est menée pendant 3 à 30 jours, par exemple pendant 10 à 30 jours, par exemple pendant 21 à 24 jours.

De préférence, la phase d'élicitation (iii) est menée à l'obscurité. De préférence, la phase d'élicitation est menée à environ 27°C. De préférence, la phase d'élicitation est menée sous agitation.

Selon un premier mode de réalisation à l'échelle du laboratoire, les cellules en suspension sont cultivées dans des récipients d'un volume d'environ 250ml, par exemple en erlenmeyer, ou bouteille de culture.

Selon un deuxième mode de réalisation à l'échelle industrielle, les cellules en suspension sont cultivées en bioréacteur agité et alimenté en air enrichi en oxygène pur. Le dispositif de culture comprend, par exemple, deux bioréacteurs qui peuvent communiquer entre eux. C'est un dispositif de culture binaire. Un bioréacteur peut être de type cuve ou de type poche. Le premier bioréacteur du dispositif binaire est le bioréacteur de propagation. Le deuxième est le bioréacteur de production. Un transfert de biomasse peut être réalisée entre le premier et le second réacteur. Ainsi, le premier réacteur dans lequel se déroule la phase de propagation alimente le second bioréacteur en biomasse pour la phase de production. A chaque transfert, le premier bioréacteur de propagation conserve un reliquat de suspension cellulaire pour relancer une étape de propagation avec du milieu de propagation frais. Il s'agit de la technique de culture en pied de cuve. Ce premier bioréacteur peut être précédé des bioréacteurs de taille inférieure pour assurer les précultures nécessaires, dans le cas d'une production à l'échelle industrielle.

Dans le même temps, le deuxième bioréacteur de production qui a reçu la biomasse du premier bioréacteur est complété par du milieu nutritif pour le servage hormonal éventuellement ou directement le milieu de production du métabolite secondaire. Ensuite le cocktail d'élicitation est introduit dans le bioréacteur de production.

Ces deux cultures en cuves sont régulées en température, la pression partielle en oxygène (pO₂) et la pression partielle en pCO₂ de la manière suivante. Les bioréacteurs sont maintenus en température par une circulation d'eau thermostatée en circuit fermé dans l'enveloppe du bioréacteur.

Une sonde à oxygène est étalonnée par saturation à l'air et renseigne en temps réel un dispositif informatisé de régulation de la pO₂ activé de manière à maintenir la pO₂ à 80% par injection d'oxygène pur stérile dans le dispositif d'aération. Ce bioréacteur est équipé également d'un dispositif de mesure en ligne du CO₂ au niveau des gaz effluents (head space) qui renseigne en temps réel un dispositif informatisé de régulation de la pCO₂ de manière à maintenir la pCO₂ à 6%. Cette dernière est faite par injection d'air atmosphérique stérile dans le dispositif d'aération en mélange avec l'oxygène. Le bioréacteur est également équipé d'un système d'agitation à pale marine tournant à vitesse constante de manière suffisante pour brasser la suspension cellulaire et éviter qu'elle ne sédimente.

Le procédé de l'invention est particulièrement avantageux puisque
- la productivité de la culture est supérieure à 2,75 mg de triptolide par litre et par jour,
- la teneur en triptolide du surnageant de culture après l'étape (iii) est supérieure à 50 mg/L
- et la teneur en triptolide en % m/m de la biomasse sèche est environ de 0,385%.

A titre de comparaison, la référence CN 101358180 A décrit une productivité volumique de 0.041 mg de triptolide par litre et par jour (0.82 mg/L en 20 jours de culture). Le procédé de la présente invention permet d'obtenir en environ 6h30 ce que le procédé de CN101358180 obtient plusieurs jours.

L'étape (iv) du procédé de l'invention consiste à extraire le triptolide du milieu de culture dans lequel il est produit.

Le tripolide peut être extrait du milieu de culture selon des méthodes bien connues de l'Homme du Métier, par exemple par extraction liquide/liquide

Cette extraction aboutit soit à du triptolide pur, soit à un extrait enrichi en triptolide.

Selon un mode de réalisation particulier l'étape (iv) est une extraction liquide/liquide par l'acétate d'isopropyle.

Un autre objet de l'invention est l'utilisation du cocktail d'élicitation tel que décrit ci-dessus pour la culture cellulaire de l'espèce *Tripterygium.*

Un autre objet de l'invention concerne un extrait enrichi en triptolide susceptible d'être obtenu par extraction du milieu de culture d'une culture *in vitro* de cellules dédifférenciées de l'espèce *Trypterigium* par le procédé de l'invention, l'extrait étant obtenu par extraction liquide/liquide dans l'étape (iv), et la teneur en triptolide du surnageant de culture après l'étape (iii) du procédé étant supérieure à 50 mg/litre.

Il est aussi décrit ici une composition dermocosmétique ou dermatologique comprenant du triptolide ou un extrait enrichi en triptolide à titre de principe actif et un ou plusieurs excipients dermocosmétiquement et/ou dermatologiquement acceptables.

Les excipients dermocosmétiquement et/ou dermatologiquement acceptables peuvent être tout excipient parmi ceux connus de l'homme de l'art en vue d'obtenir une composition pour l'application topique sous forme de crème, d'une lotion, d'un gel, d'une pommade, d'une émulsion, d'une microémulsion, d'un spray, etc.

La composition dermocosmétique ou dermatologique peut en particulier contenir des additifs et aides à la formulation, tels que des émulsionnants, des épaississants, des gélifiants, des fixateurs d'eau, des agents d'étalement, des stabilisants, des colorants, des parfums et des conservateurs.

Il est également divulgué une composition dermatologique comprenant du triptolide ou un extrait enrichi en triptolide à titre de principe actif et un ou plusieurs excipients cosmétiquement et/ou pharmaceutiquement acceptables, pour son utilisation dans le traitement des troubles inflammatoires cutanés, préférentiellement le prurit, l'eczéma, la dermatite atopique et le psoriasis.

Il est également divulgué l'utilisation d'une composition dermatologique comprenant du triptolide ou un extrait enrichi en triptolide à titre de principe actif et un ou plusieurs excipients dermocosmétiquement et/ou dermatologiquement acceptables, pour la fabrication d'un médicament destiné au traitement des troubles inflammatoires cutanés, préférentiellement le prurit, l'eczéma, la dermatite atopique et le psoriasis.

Il est aussi décrit un extrait enrichi en triptolide pour son utilisation en tant que médicament.

Il est aussi décrit le Triptolide ou un extrait enrichi en triptolide pour son utilisation dans le traitement des troubles inflammatoires cutanés, préférentiellement le prurit, l'eczéma, la dermatite atopique et le psoriasis.

Il est aussi décrit l'utilisation du Triptolide ou d'un extrait enrichi en triptolide pour la fabrication d'un médicament destiné au traitement des troubles inflammatoires cutanés, préférentiellement le prurit, l'eczéma, la dermatite atopique et le psoriasis.

Il est aussi décrit l'utilisation dermocosmétique du Triptolide ou d'un extrait enrichi en triptolide.

Les figures et exemples suivants illustrent l'invention sans en limiter la portée.
Figure 1 : Exemple de culture en bioréacteurs (système binaire). Propagation de biomasse dans le fermenteur A, transfert du milieu vers le fermenteur de production B, sevrage, élicitation, et récolte de la biomasse quelques semaines après.
Figure 2 : Exemple de cinétique de production du triptolide (PG 490) par élicitation dans le fermenteur B.
Figure 3: Bioréacteur à usage unique Wave (GE Healthcare Biosciences) type Rocker 20/50EHT équipé d'un mélangeur de gaz Air et Oxygène pur (02MIX), d'un système de mesure de la pression partielle en oxygène dissous pO₂ (DOOPT20 + DOOPT-PROBE) et du plateau pour installer les Cell bags de 10 et 20 Litres (kit 20EHT) régulant la température à 27°C.
Figure 4: Cinétique de culture de production du triptolide en bioréacteur à usage unique de type Wave. Suivi de paramètres physico-chimiques : pH, pression partielle d'Oxygène dissout pO₂, consommation en Saccharose, évolution de la biomasse sèche (DW) et teneur en triptolide dans le surnageant de culture.
Figure 5 : dosage par HPLC permet de déterminer la concentration en Triptolide dans l'extrait
Figure 6 : Test d'inhibition de NFκB par l'extrait CCV
Figure 7 : Effet de l'extrait CCV (IB0-18.134) sur la production de NO₂⁻ par des cellules RAW264.7 stimulées avec 1 µg/ml de LPS Figure 8 : Effet de l'extrait CCV sur l'inhibition de PAR 2
Figure 9 : Effet des composés sur le modèle Dermatite atopique
Figure 10 : Effet des composés sur le modèle Psoriasis

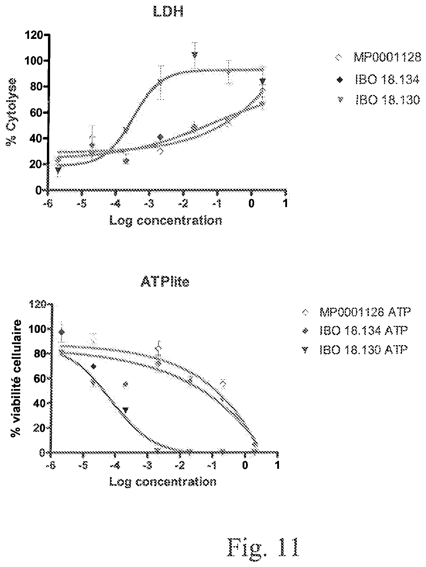

Figure 11 : Tests de cytotoxicité (Dosage ATPlite et Dosage LDH)

### Exemple 1 : Protocole de dédifférenciation cellulaire :

Les cals sont obtenus à partir d'explants de feuilles de *Tripterygium wilfordii.*

Les explants sont stérilisés à l'éthanol à 70% puis à l'hypochlorite de Na à 2,5% de chlore actif, puis rincé à l'eau déminéralisée stérile. Optionnellement, un lavage avec du péroxyde d'hydrogène à 7% avant le rinçage par l'eau déminéralisée stérile est possible

Les feuilles sont découpées en morceaux, par exemple en carrés d'environ 8 - 10 mm de côté. Les explants foliaires sont déposés sur un milieu gélosé d'induction de dédifférenciation (milieu MSO) et de repiquage.

La composition du milieu de dédiférenciation est la suivante :
Macroéléments : NH₄NO₃ à 1650 mg/L, KNO₃ à 1900 mg/L, CaCl₂.2H₂O à 440 mg/L, MgSO₄.7H₂O à 370 mg/L, KH₂PO₄ à 170 mg/L,
Microéléments : KI à 0,83 mg/L, H₃BO₃ à 6,2 mg/L, MnSO₄.4H₂O à 22,3 mg/L, ZnSO₄.H₂O à 6,61 ou 8,6 mg/L, Na₂MoO₄.2H₂O à 0,25 mg/L, CuSO₄.5H₂O à 0,025 mg/L, CoCl₂.6H₂O à 0,025 mg/L, FeSO₄.7H₂O à 27,8 mg/L, Na₂EDTA.2H₂O à 37,3 mg/L,
Vitamines : myo-Inositol à 100 mg/L, acide Nicotinique à 0,5 mg/L, Pyridoxine-HCl à 0,5 mg/L, Thiamine-HCl à 0,5 mg/L, Glycine à 2g/L
Source de carbone : Saccharose à 30 g/L,
Hormones : Kinétine à 0,1 mg/L, Acide 2-4 dichlorophénoxyacétique (2,4D) à 0,5 mg/L, Acide Naphthalène acétique (NAA) à 1 mg/L

Le milieu de dédifférenciation est gélifié par addition d'agar à 8-12 g/L,son pH est ajusté à pH 6 ± 0,5 avant un autoclavage de 20 min à 121°C. Les boîtes de pétri contenant les explants sont incubées à l'obscurité à 27-28°C.

Les cals obtenus sont détachés de l'expiant foliaire et déposés sur de nouvelles géloses de dédifférenciation. Le repiquage des cals est réalisé tous les mois sur le même milieu gélosé.

### Exemple 2 : Formulation des milieux de culture et de propagation :

Dès l'obtention de cals friables, après quelques mois de repiquage, ceux-ci sont transférés dans le milieu de culture liquide, optimisé pour la propagation de la suspension cellulaire.

La mise en suspension cellulaire est réalisée en déposant environ 40 g de cals friables dans un Erlen de 200 ml contenant le milieu de propagation, incubation pendant une semaine sur une table d'agitation à 100 RPM à l'obscurité à 27-28°C. Le surnageant cellulaire est récolté à la pipette laissant les amas de cals résiduels. La suspension cellulaire obtenue est cultivée pendant 15 jours puis propagée par dilution au 1/5^{ème} dans du nouveau milieu tous les 15 jours. Les suspensions cellulaires cultivées sur les milieux de propagation TW2H6 ont donné naissance à la lignée NS.

Le milieu de propagation a, par exemple, la composition indiquée ci-dessous :
Macroéléments : NH₄NO₃ à 1650 mg/L, KNO₃ à 2500 mg/L, CaCl₂.2H₂O à 440 mg/L, MgSO₄.7H₂O à 370 mg/L, KH₂PO₄ à 130 mg/L,
Microéléments : KI à 0,41 mg/L, H₃BO₃ à 6,2 mg/L, MnSO₄.4H₂O à 22,3 mg/L, ZnSO₄.H₂O à 7,5 mg/L, Na₂MoO₄.2H₂O à 0,25 mg/L, CuSO₄.5H₂O à 0,025 mg/L, CoCl₂.6H₂O à 0,025 mg/L, FeSO₄.7H₂O à 19,85 mg/L, Na₂EDTA.2H₂O à 26,64 mg/L,
Vitamines : myo-Inositol à 50 mg/L, acide Nicotinique à 0,25 mg/L, Pyridoxine-HCl à 0,25 mg/L, Thiamine-HCl à 0,25 mg/L.
Hormones: Kinétine à 0,083 mg/L, Acide 2-4 dichloro-phenoxyacetic (24D) à 0,575 mg/L, Acide Naphthalene acétique (NAA) à 0,350 mg/L.
Sources de C : Saccharose à 30 g/L.

Le pH du milieu est ajusté à pH 6 ± 0,5 avant un traitement de stérilisation approprié par exemple, autoclave à 121°C pour une durée minimale de 20 minutes ou par filtration stérilisante sur 0,2 µm.

Le taux de remplissage des Erlenmeyers est de 20-40% et le taux d'inoculation par transfert de suspension cellulaire est de 20-25% du volume soit environ 50-100 g/L env. de biomasse fraîche. La culture est menée ainsi pendant 15 jours à l'obscurité à 27-28°C sous agitation orbitale de 110-120 RPM (rotation par minute). A ce stade la biomasse est présente à une concentration pouvant aller jusqu'à 320-350 g/L environ de biomasse fraiche.

La propagation peut également avoir lieu dans un milieu de dédifférenciation.

### Exemple 3 : production de Triptolide en Erlenmeyer

La production en erlen meyer se décompose ensuite en trois phases :
- culture cellulaire sur milieu de propagation pendant 15 jours
- sevrage hormonal pendant 7 jours
- production de triptolide par le biais d'une élicitation pendant environ 20 jours.

A l'issue d'une culture de propagation de la biomasse en Erlenmeyer de 15 jours, comme indiqué ci-dessus, celle-ci est mise à sédimenter de manière à pouvoir retirer partiellement le surnageant, par exemple1/3 du volume total de la suspension, et le remplacer par le même volume de milieu de sevrage hormonal, par exemple le milieu de sevrage hormonal décrit ci-dessous. Ce milieu de sevrage a pour objectif d'éliminer les résidus de l'hormone de croissance 2,4-D, qui est un inhibiteur de la production de triptolide, présent dans le milieu de propagation. La composition du milieu de sevrage hormonal est le suivant :
Macroéléments : NH₄NO₃ à 2 g/L, KNO₃ à 3 g/L, CaCl₂.2H₂O à 440 mg/L, MgSO₄.7H₂O à 370 mg/L, KH₂PO₄ à 43 mg/L, Pyruvate de sodium 2 g/L.
Micro-éléments : KI à 0,41 mg/L, H₃BO₃ à 6,2 mg/L, MnSO₄.4H₂O à 22,3 mg/L, ZnSO₄.H₂O à 7,5 mg/L, Na₂MoO₄.2H₂O à 0,25 mg/L, CuSO₄.5H₂O à 0,025 mg/L, CoCl₂.6H₂O à 0,025 mg/L, FeSO₄.7H₂O à 19,85 mg/L, Na₂EDTA.2H₂O à 26,64 mg/L,
Vitamines : myo-Inositol à 50 mg/L, acide Nicotinique à 0,25 mg/L, Pyridoxine-HCl à 0,25 mg/L, Thiamine-HCl à 0,25 mg/L, Glycine à 1g/L
Hormones : Kinétine à 1,1 mg/L, Acide Indole-butyrique(IBA) à 2 mg/L.
Source de Carbone : Saccharose à 30 g/L

Le pH est ajusté à pH 6 ± 0,5 avant un autoclavage de 20 min à 121°C.

Le sevrage est mené ainsi pendant 7 jours à l'obscurité à 27-28°C sous agitation orbitale de 110-120 RPM.

Une fois l'étape de sevrage réalisée, la biomasse est essorée par filtration sur buchner et inoculée dans du milieu de sevrage hormonal neuf à raison de 100 à 200 g/L environ de biomasse fraîche.

Le cocktail d'élicitation est introduit, par exemple à l'aide de solutions mères réalisées dans du diméthylsulfoxyde, dans le milieu de culture. La composition du cocktail éliciteur est la suivante : Acide Abscissique (ABA) à 1,25 mg/L, Benzyl-aminopurine (BAP) à 0,7 ou à 3 mg/L, Acide 5-Chloro Salicylique (5-Chloro SA) à 3 ou 5 mg/L, Acide Acetyl-Salicylique (ASA) à 33 ou 45 mg/L, Methyl-Jasmonate (MeJA) à 22,4 mg/L , Farnesol (F-OH) à 19 ou 30 mg/L et Géraniol à 23 mg/L.

La production de triptolide est menée ainsi pendant 10 à 21 jours à l'obscurité à 27-28°C sous agitation orbitale de 120 RPM. A l'arrêt de culture, on filtre le milieu pour récupérer le surnageant limpide coloré sombre qui contient la majorité de triptolide.

La concentration en triptolide dans le surnageant de culture est comprise entre 50 et 70 mg/litre, par exemple entre 45 et 65 mg/L de milieu de culture.

### Exemple 4 : production de triptolide en Bioréacteurs de type cuve agitée

La production de triptolides en bioréacteur se décompose en trois phases:
1. Culture cellulaire sur milieu de propagation pendant 15 jours
2. Sevrage hormonal pendant 7 jours
3. Production de triptolides par le biais d'une élicitation pendant 21 jours

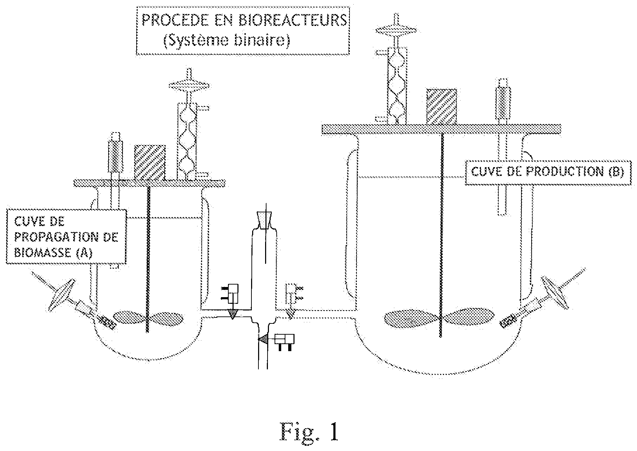

Culture cellulaire et propagation sur milieu de propagation :
A l'issue d'une culture de propagation de la biomasse en Erlenmeyer de 15 jours, comme indiqué ci-dessus, celle-ci est utilisée comme inoculum pour la culture en bioréacteur de 10 L (réacteur A). Voir Figure 1 du système binaire.

L'inoculum de 2 L est déversé dans le bioréacteur de propagation (A). Ce bioréacteur contient 8 L de milieu de propagation à 27,5°C.

Une sonde à oxygène est étalonnée par saturation à l'air et renseigne en temps réel un dispositif informatisé de régulation de la pO₂ activé de manière à maintenir la pO₂ à 50 - 80%. Ce bioréacteur est équipé également d'un dispositif de mesure en ligne du CO₂ au niveau des gaz effluents (head space) qui renseigne en temps réel un dispositif informatisé de régulation de la pC02 de manière à maintenir la pCO₂ à 6 - 8%. Le bioréacteur est également équipé d'un système d'agitation à pale marine tournant à 75 RPM de manière à éviter la sédimentation des cellules au fond du réacteur.

La culture est maintenue dans ces conditions physico-chimiques pendant 15 jours de manière à atteindre une densité cellulaire de l'ordre de 320 g/L de biomasse fraîche.

Ensuite ce bioréacteur est raccordé stérilement à un autre bioréacteur nommé bioréacteur de production (B). On effectue le transfert de l'équivalent de 1700 g de biomasse fraiche (environ 5,3 L de vol) de A vers B.

Le bioréacteur de propagation A garde un reliquat de 2 L de suspension auquel est ajouté un volume de 8 L de milieu de propagation déversé de manière à relancer la propagation cellulaire.

### Sevrage et Production sur milieu de sevrage :

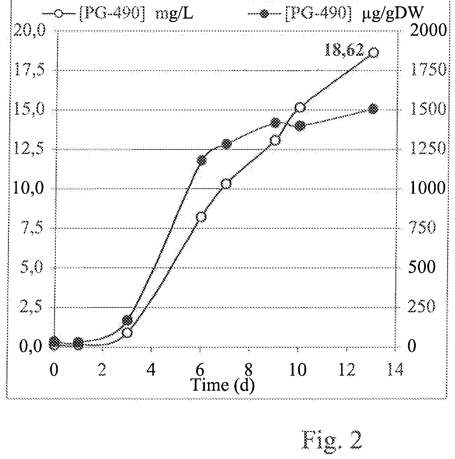

Dans le même temps, le bioréacteur de production B est complété par du milieu de sevrage de manière à atteindre un volume de 10 L et une densité cellulaire de 170 g/L . La culture dans le bioréacteur de production est maintenue en l'état pendant 4 à 6 jours de manière à sevrer complètement la biomasse de traces des hormones de croissance, de type auxine comme le 2,4D présentes dans le milieu de propagation. Ensuite le cocktail d'éliciation est introduit dans le bioréacteur de production B. La production de triptolide est menée ainsi pendant 15 à 21 jours. La Figure 2 montre un exemple de suivi cinétique de production de Triptolide après élicitation (à t = 0) dans la cuve B. En fin de culture, on récupère le surnageant de culture par une étape de pré filtration (15 - 20 µm) suivi par la filtration à 0,2 µm. On obtient une solution limpide. La concentration en triptolide obtenue est de 20 à 35mg/L dans le milieu extracellulaire.

Ce type de culture binaire peut être réaliser à des échelles en fermenteur de taille supérieures (> 100 L en volume).

### Exemple 5 : Production de triptolide en bioréacteurs en poches à usage unique de type WAVE

Un autre procédé utilisant de manière avantageuse un équipement très simplifié et peu coûteux permet d'avoir un rendement comparable au système décrit précédemment. Il s'agit de réacteurs à usage unique qui ne nécessitent pas d'entretien et de nettoyages lourds comme dans le cas des fermenteurs traditionnels en INOX. Le réacteur agité est couramment utilisé dans la culture des cellules mammifères en suspension. L'exemple illustré est décrit pour réacteurs de type WAVE, par exemple, de marque GE Healthcare Biosciences, pour des volumes de 10 L ou 20 L, mais la méthode peut être adaptée et appliquée à des volumes supérieurs et de matériels issus d'autres fabricants.

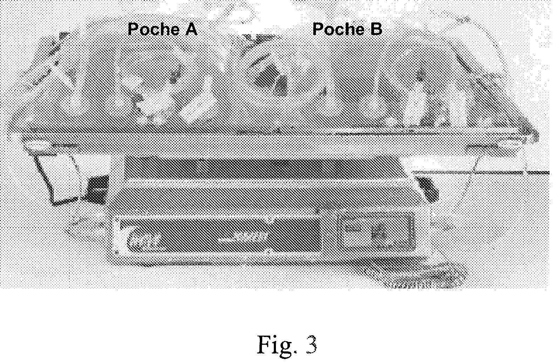

Le système binaire décrit précédemment pour des bioréacteurs traditionnels de laboratoire en verre ou industriel en inox est appliqué de la même manière avec les 2 poches WAVE. Voir illustrations sur Figure 3.

### Propagation :

Le bioréacteur Wave A (10L) placé sur son support est rempli du milieu nutritif par filtration stérilisante en ligne et gonflé par de l'air. Il est ensuite inoculé grâce à une pré-culture réalisée en erlenmeyer agité pendant 15 jours en milieu de propagation, en incubateur agité.

Le bioréacteur est incubé selon les conditions suivantes :
- angle de basculement : 6-8°
- fréquence de basculement : 16- 20 RPM
- débit d'aération : 0,1 - 0,15 L /mn d'air enrichi à 50% en oxygène pur
- T = 27°C
- Durée = 14 Jours

### Elicitation et Production :

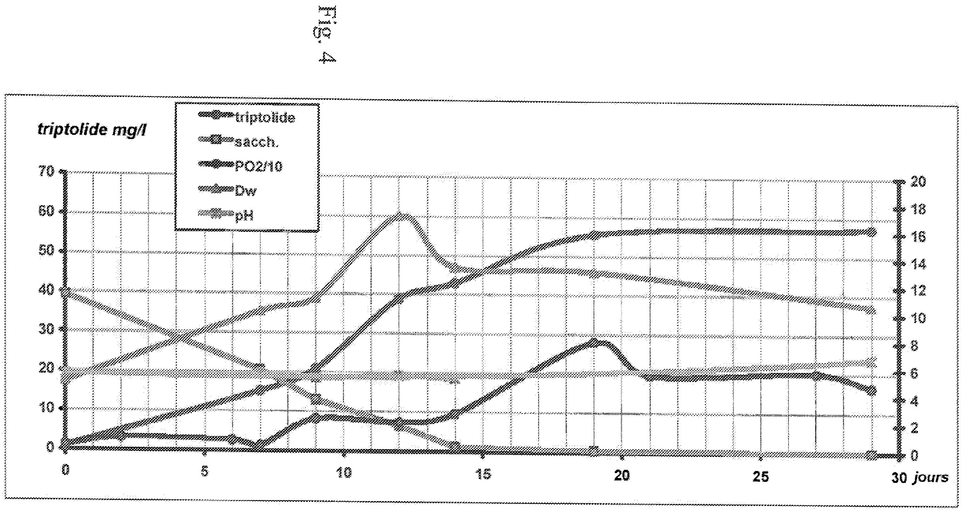

Un volume d'environ 500 ml de culture de la poche A est transféré dans la poche B (10 L) placée à côté de la poche A du plateau. Le reliquat de la poche A (environ 2 L) est dilué par 2 L de milieu de sevrage concentré 2 fois On poursuit l'agitation à T = 27°C. La culture en phase d'élicitation est suivie par la mesure de certains paramètres voir Figure 4. La concentration maximale de triptolide est de 55 mg par litre de surnageant de culture, après 19 jours d'incubation. La vitesse de production du triptolide est d'environ 2,87 mg/L de surnageant acellulaire et par jour de culture. La cinétique de production de triptolide commence à s'infléchir peu de temps après avoir consommé la quasi-totalité du saccharose disponible. En fin de culture, on récupère le surnageant de culture suivie par une étape de pré filtration (15 - 20 µm) et une 2^{e} étape de filtration à 0,2 µm. On obtient une solution limpide colorée (jaune ou mauve rose).

### Exemple 6: Production de Triptolide en bioréacteur Wave

Température d'incubation : +27°C pour toutes les phases (croissance cellulaire et élicitation en fioles et bioréacteur).

### Préculture :

Elle est réalisée en fiole agitée de 1 litre contenant 400ml de milieu de propagation TW2H6, qui a été inoculée par 100 ml d'une culture d'environ 15 jours.

**Tableau 1 : Caractéristiques de la pré culture à terme**

| ***t0* + *15j*.** | ***contenant*** | ***culture NS*** | ***pH*** | ***Fw (g*/*l)*** | ***Dw (g*/*l)*** | ***sacch. (g*/*l)*** | ***triptolide (mg*/*l)*** |
|---|---|---|---|---|---|---|---|
| **NS P6 G** | *erlen verre 1l* | *beige* | *5,6* | 131 | 7,2 | 10,4 | **2,9** |

### Culture en Bioréacteur Wave :

L'inoculum de 500 ml est transféré dans le bioréacteur à usage unique installé et rempli du milieu nutritif stérile, à l'aide de connexion stérile. Celle-ci est incubée sur le rocker selon les conditions suivantes :
- angle de basculement : 6-8°
- fréquence de basculement : 16-18 RPM
- débit d'aération : 0,1-0,15 litre / minute d'air enrichi à 50% en oxygène pur
- durée de la culture : environ 14 jours

**Tableau 2 : Caractéristiques de la culture à terme**

| ***t0*+*14j.*** | ***contenant*** | ***culture NS*** | ***pH*** | ***Fw(g*/*l)*** | ***Dw(g*/*l)*** | ***sacch (g*/*l)*** | ***triptolide (mg*/*l)*** |
|---|---|---|---|---|---|---|---|
| **EW4-NSP7** | *poche 10l* | *beige kaki clair* | *5,8* | 253 | 9,7 | 0,5 | 1,1 |

### Elicitation en Bioréacteur Wave :

Un volume de 2 litres de cette culture est élicité par dilution dans 2 litres de milieu de sevrage contenant le cocktail d'élicitation concentré 2 fois, l'incubation est poursuivie dans les conditions suivantes :
- angle de basculement : 6 à 7°
- fréquence de basculement : 17 à 21 RPM
- débit d'aération : 0,1 à 0,15 litre / minute d'air enrichi à 50% en oxygène

### Résultats :

La croissance cellulaire et la production du métabolite par élicitation a été conduite dans des bioréacteurs à usage unique, par exemple une poche de type Wave.

Le suivi de la culture en élicitation est représenté à la Figure 4.

Durant la phase d'élicitation, certains paramètres sont suivis :
- La masse sèche en suspension DW : elle augmente de 5g/litre à 13g/litre en 10 jours, stagne pendant 10 jours, puis décroît légèrement à 10g/litre en 10 jours, probablement du à un début de lyse cellulaire.
- Le pH est assez stable pendant 20 jours (valeur autour de 5,5), puis augmente progressivement vers 7 sur les 10 derniers jours de la culture. Cette élévation de pH pourrait être attribuée à la lyse cellulaire, et au relargage du contenu cytoplasmique.
- La pression partielle en oxygène p02 (% de la saturation du milieu en oxygène) reflète la quantité d'oxygène dissoute dans le milieu au moment de la mesure, donc la résultante entre la quantité totale que peut contenir le milieu (= la saturation) et la quantité d'oxygène consommée par les cellules.
- La teneur en saccharose totale décroit pendant les 15 premiers jours à une vitesse de 2,7g par litre et par jour
- La teneur en triptolide croit jusqu'à une teneur de l'ordre de 55-56mg/litre en 19 jours, puis reste pratiquement stable jusqu'à la fin de la culture.

### Exemple 7: Production d'un extrait enrichi en Triptolide IB0.18.134 par extraction liquide/liquide du surnageant de culture de Trypterigium wilfordii

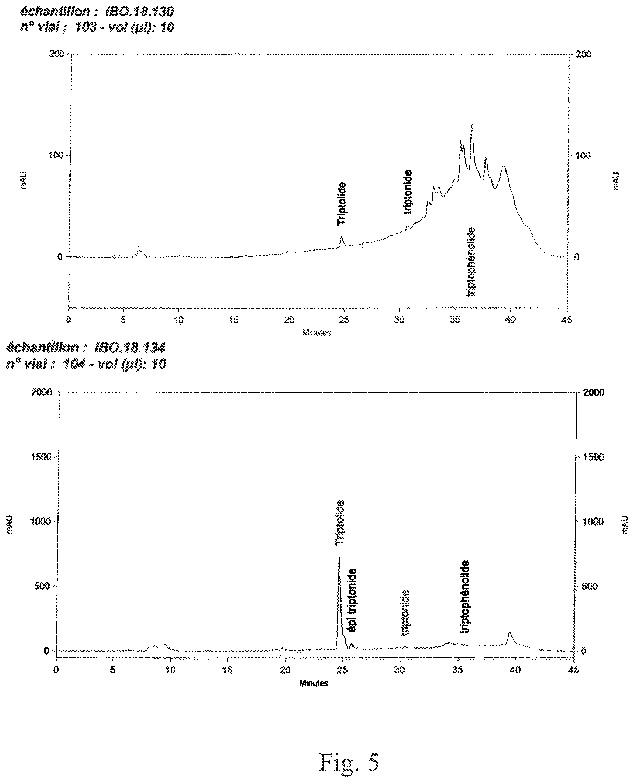

On extrait d'environ 30 L de surnageant de culture contenant le Triptolide obtenu à l'Exemple 6 par un volume d'Acétate d'Isopropyle (2 fois consécutives). Concentration et séchage des phases organiques à l'évaporateur rotatif. On obtient 650 mg de matière sèche de couleur beige jaune. Le dosage par HPLC a permis de déterminer la concentration en Triptolide dans l'extrait : 195 mg de Triptolide contenu dans les 0,65g de poudre récupérée soit encore 0,3 g de Triptolide/g d'extrait sec.
(voir Figure 5)

### Exemple 8 (exemple comparatif): Production d'un extrait enrichi en Triptolide de racines IBO.18.130 :

Les racines de *Trypterigium wilfordii* sont écorcées, séchées et broyées. Elles sont ensuite extraites par de l'éthanol 90%. Une fois concentré, l'extrait est soumis à une extraction liquide/liquide avec du 1,2-dichloro-éthane. La phase chlorée est lavée avec une solution basique (NaOH), concentrée et adsorbée sur silice. Cet extrait brut sur silice est stocké à -20°C.

Extraction méthanolique des racines de *Trypterigium wilfordii* adsorbé sur silice

200g d'extrait brut sur silice dans 1 litre de méthanol (1 seule extraction) sous agitation magnétique à température ambiante pendant 1 h. Séchage de la phase méthanolique à l'évaporateur rotatif. On obtient 25g de matière sèche de couleur brun-orangé. Le dosage par HPLC permet de déterminer la concentration en Triptolide dans l'extrait : 90 mg de Triptolide contenu dans les 25g de poudre récupérée soit encore 0,0036g de Triptolide/g de l'extrait sec.
(voir Figure 5)

Les exemples suivants 9 à 12 comparent :
1) Les extraits de culture de cellule végétale (CCV) de l'Exemple 7 par rapport au Triptolide pur (à concentration identique en TRP) :
   ▪ inhibition de la transcription de NFκB (inhibition des réponses pro inflammatoires et inflammatoires)
   ▪ inhibition de la production des nitrites (NO₂)
   ▪ inhibition de récepteur PAR2.
2) les extraits de racine (R) de l'Exemple 8 et les extraits de culture cellule végétale (CCV) de l'Exemple 7 en termes d'activités pharmacologiques (à concentration identique en TRP) :
   ▪ inhibition de gènes inflammation (modèle Dermatite atopique)
   ▪ inhibition de gènes inflammation (modèle Psoriasis)
3) la cytotoxicité *in vitro* des 2 extraits R et CCV a également été comparée

### Exemple 9 : Test d'inhibition de NFκB par l'extrait CCV

Le facteur de transcription NF-κB contrôle l'expression d'un grand nombre de gènes impliqués dans la régulation de la réponse inflammatoire. Certains stimuli pro-inflammatoires tels que le TNF-α (Tumor Necrosis Factor α) conduisent à l'activation de NF-κB c'est-à-dire à sa translocation nucléaire. Par conséquent, NF-κB va induire la transcription des gènes pro-inflammatoires codant des cytokines, des chémokines, des molécules d'adhésion, des facteurs de croissance et des enzymes inductibles telles que la cyclooxygénase 2 (COX2) et la nitric oxide synthase (iNOS). NF-κB joue un rôle clé dans l'initiation et l'amplification de la réponse inflammatoire. Certaines maladies inflammatoires chroniques de la peau, telles que la dermatite atopique ou le psoriasis, sont caractérisées par une dérégulation du niveau d'expression des médiateurs de l'inflammation exprimés par les kératinocytes. L'activité anti-inflammatoire du TRP pur comparée à celle des extraits de culture de cellule végétale (IBO 18.134) à des doses équivalentes en TRP est évaluée.

### Résultats :

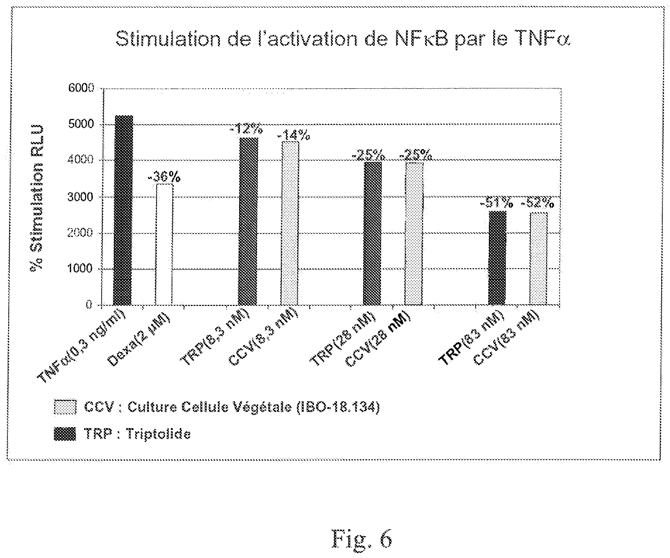

La figure 6 représente l'inhibition des différents extraits sur l'activation de NF-κB suite à une stimulation TNF-α dans les cellules de Kératinocytes HaCat. Comme contrôle positif, la Dexaméthasone (2µM), qui inhibe de 36% l'activation de NF-κB a été utilisée. Le Triptolide pur inhibe de façon dose dépendante de 8nM à 83 nM.

A concentration identique en Triptolide à 8 nM, à 28 nM et à 83 nM, l'inhibition de NFκB du CCV et du TRP sont équivalentes.

### Exemple 10 : Effet de l'extrait CCV (IBO-18.134) sur la production de NO₂⁻ par des cellules RAW264.7 stimulées avec 1 µg/ml de LPS

L'objectif de cette étude était de comparer l'activité anti-inflammatoire de l'extrait CCV (IB-134) à celle du triptolide pur.

Pour ce faire, le test retenu était la production de NO₂⁻ par des cellules RAW264.7 stimulées avec du LPS (Lipopolysaccharides).

Brièvement, les cellules RAW264.7 (macrophages murins) ont été ensemencées à 1,4 x 10⁵ cellules/cm². Au bout de 24h, les cellules ont été incubées 1 h avec différentes concentrations des produits à tester puis stimulées durant 24h avec 1µg/ml de LPS. La concentration de NO₂⁻ a été estimée dans les surnageants de culture à l'aide du réactif de Griess.

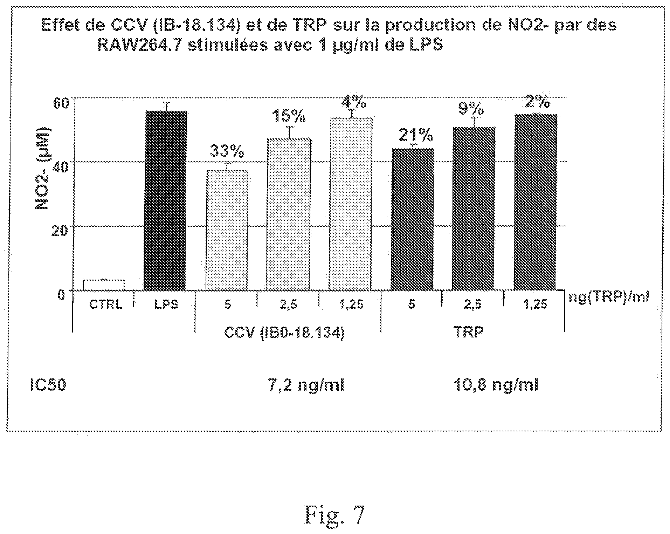

Les résultats (Figure 7) illustrent la production de NO₂⁻ obtenue dans les différentes conditions précisées plus haut. Le pourcentage d'inhibition calculé par rapport au contrôle LPS figure dans chaque histogramme. Enfin, l'IC50 a été calculé à partir de ces valeurs (en gras et bleu).

Les données obtenues montrent que, dans les conditions testées à concentration identique de TRP, l'extrait CCV (IB-18.134) et le TRP inhibent la production de nitrites induite par le LPS, l'inhibition est légèrement plus forte en faveur du CCV.

La comparaison des IC50 montre que le CCV est supérieur au TRP. L'extrait CCV se révèle plus actif que le triptolide pur pour l'inhibition de NO₂⁻.
(voir Figure 7)

### Exemple 11 : Effet de l'extrait CCV sur l'inhibition de PAR 2

Le récepteur « Protease-Activated Receptor-2 (PAR-2) » est associé à la physiopathologie de plusieurs maladies impliquant des réponses inflammatoires.

Le PAR-2 appartient à la superfamille des récepteurs à 7 domaines transmembranaires couplés aux protéines G, mais possède une voie d'activation unique.

En effet, le PAR-2 est activé par des sérine-protéases telles que la trypsine, la tryptase, les facteurs Xa et VIIa. Le clivage par ces protéases de la partie extracellulaire du récepteur expose un nouveau domaine amino-terminal (SLIGKV) qui agit comme un ligand « attaché » au récepteur : il se fixe sur lui-même au niveau de la boucle extra-cellulaire 2 et s'auto-active.

Le PAR-2 est exprimé par les différents types cellulaires de la peau :
kératinocytes, cellules myoépithéliales des glandes sudoripares, follicule pileux, cellules dentritiques-like du derme et les cellules endothéliales de la lamina propria et du derme. Les mélanocytes n'expriment pas ce récepteur bien que le PAR-2 joue un rôle important dans la pigmentation en favorisant le transfert de mélanine des mélanocytes vers les kératinocytes.

Les sérine-protéases générées par l'épiderme exercent des effets chimiotactiques induisant le recrutement des leucocytes dans la peau. Elles sont également impliquées dans la régulation de l'homéostasie, de la mitogenèse et de la différenciation épidermiques et modulent la fonction barrière de la peau. De plus, les sérine-protéases contribuent à la physiopathologie de maladies cutanées liées à l'inflammation, la défense de l'hôte, la cancérogenèse, la fibrose et la stimulation nerveuse.

Les propriétés physiologiques et physiopathologiques cutanées des sérine protéases seraient en partie liées aux récepteurs PARs. En effet, les récepteurs PAR-2 sont sur-exprimés dans l'épiderme, le derme et les vaisseaux des maladies inflammatoires de la peau comme la dermatite atopique, le lichen plan et le psoriasis. Les récepteurs PAR-2 joueraient également un rôle dans le développement du prurit chez des patients atteints de dermatite atopique.

L'activation de PAR-2 par une protéase de type trypsine induit la production d'IL-8 à partir de kératinocytes (HaCaT). Plus récemment, il a été démontré que l'IL-8, chémokine chimio-attractive pour les leucocytes, permettrait l'infiltration de neutrophiles dans l'épiderme de patients atteints de Psoriasis vulgaris.

La signalisation intracellulaire du récepteur PAR-2 est sous-tendue pour partie par une mobilisation de calcium intra-cellulaire.

Nous nous sommes donc proposé d'évaluer l'activité anti-PAR-2 de l'extrait CCV (IB0.18.134) et du Triptolide, sur des kératinocytes humains issus d'une lignée (HaCaT), en mesurant l'influx de calcium intracellulaire induit suite à la stimulation spécifique des récepteurs PAR-2 par la trypsine.

*In vitro,* à l'échelle cellulaire, la stimulation des récepteurs PAR-2 par la trypsine conduit à une mobilisation de calcium intra-cellulaire détectée à l'aide d'une sonde fluorescente.

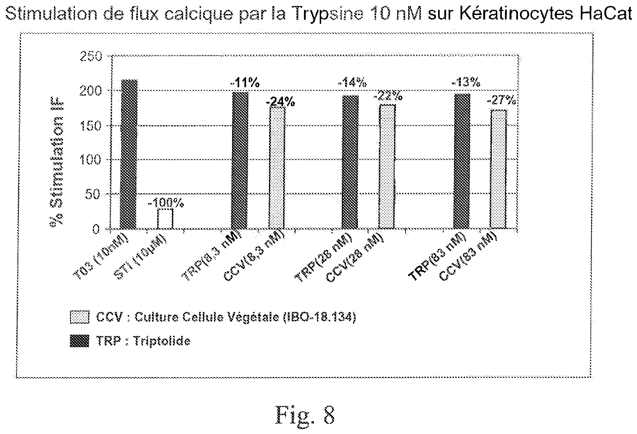

On constate que pour une concentration identique en Triptolide, une inhibition de PAR 2 modérée des 2 produits testés avec toutefois une inhibition plus marquée pour la CCV dans ces concentrations testées.
(voir Figure 8)

### Exemple 12 : Evaluation dans le modèle Dermatite atopique et le modèle Psoriasis

Comparaison de l'activité anti-inflammatoire et apaisante du triptolide obtenu à partir d'un extrait de racine (IB0.18.130) et celle d'un extrait CCV (IBO.18.134).

Cette évaluation a été recherchée dans le cadre de l'induction d'un phénotype de type dermatite atopique et de type psoriasis dans des kératinocytes épidermiques humains normaux. Plus particulièrement, l'effet de ces composés a été analysé par PCR array (puces à ARN) sur l'expression de 2 panels de 32 gènes (ARNm) sélectionnés pour leur importance dans l'inflammation des kératinocytes et plus précisément pour leur implication dans la dermatite atopique ou le psoriasis.

L'effet de ces composés a été étudié :
- d'une part dans des kératinocytes présentant un phénotype de dermatite atopique après stimulation par du poly (I:C) + association de cytokines de types Th1 (TNF-a) et Th2 (IL4 + IL13)
- d'autre part dans les kératinocytes présentant un phénotype de psoriasis après stimulation par une association de cytokines (IL17 + OSM + TNF-α)

### MATERIEL ET METHODES

### 1. Les extraits

Les extraits ont été solubilisés dans du DMSO à une solution mère de 200 mg/ml exprimée en concentration en triptolide pur. Cette concentration est imposée par la solubilité de l'extrait de racine qui demande une attention particulière (dissolution à température ambiante sous agitation douce).

Les composés ont été solubilisés extemporanément pour les tests pharmacologiques qui ont été réalisés pour les 2 extraits à 2,5 ng/ml en équivalence concentration en triptolide pur.

### 2. Type cellulaire

Kératinocytes épidermiques humains normaux (NHEK). Les cellules sont amplifiées dans des conditions standard de culture.

### 3. Pharmacologie

### 3.1 Méthodologie

Les cellules NHEK sont ensemencées et cultivées en milieu de culture Keratinocyte-SFM. Le milieu de culture est remplacé par du milieu contenant ou non (témoin) les extraits à l'essai. Après une "pré-incubation" de 1 h le mélange d'inducteur de l'inflammation, on ajoute le mélange (Polyl:C, IL4, IL13, TNF) dans le cas de la Dermatite atopique. Dans le cas du Psoriasis le mélange (IL17, Oncostatin M, TNF) est ajouté.

Un contrôle sans inducteur et sans composé est également réalisé en parallèle, nous permettant de valider le modèle induit (NHEK vs NHEK + inducteurs).

Toutes les conditions ont été réalisées en n=2.
L'ARN est extrait des cellules après une incubation de 24 heures avec le mélange d'inducteur +/- les extraits à 2,5 ng/ml d'équivalent triptolide pur.

### 3.2 Analyse de l'expression différentielle par RT-QPCR

Après extraction des ARNs totaux et synthèse des ADNc, 32 gènes spécifiques de la dermatite atopique et 32 gènes spécifiques du psoriasis sont analysés en PCR quantitative.

### • Gènes quantifiés

Les listes des gènes caractéristiques d'un phénotype DA et d'un phénotype psoriatique qui ont été quantifiés sont données, respectivement, dans les Tableaux 1 et 2.

### RESULTATS

### 1. Effets des composés sur des kératinocytes présentant un phénotype dermatite atopique

### 1.1 Validation de l'expérience

Le traitement des kératinocytes par l'association de (Poly (I:C) et de cytokines de type Th1/Th2 (IL4, IL13, TNF) a clairement induit un phénotype de type dermatite atopique en induisant l'expression de différents gènes caractéristiques impliqués dans la pathologie.

En effet, une augmentation d'expression du marqueur d'immunité innée S100A7, des cytokines (TSLP, IL1A, IFN1A) et de la plupart des chimiokines étudiées (CCL3, CCL5, CCCL7, CCL20, CCL22, CCL27 et IL8) a été observée.

Ces effets étaient accompagnés d'une augmentation des marqueurs de régulation transcriptionnelle RARRES3 et BCL3. Parallèlement une inhibition des marqueurs impliqués dans la différentiation des kératinocytes (KRT10, FLG, IVL et LASS6) est observée.

### 1.2 Effets des composés sur le modèle Dermatite atopique

Les 2 composés ont été testés à la dose de 2,5 ng/ml (équivalent en triptolide pur).

### Extrait de racine (IB0.18.130)

L'extrait de racine présente un effet anti-inflammatoire modéré sur ce modèle en inversant les effets du mélange pro-inflammatoire. En effet l'expression du marqueur d'immunité innée S100A7, des chimiokines (CCL3, CCL5 et IL8) et du marqueur de stress oxydatif (HMOX1) a été réprimé alors que l'expression du marqueur impliqué dans la différentiation des kératinocytes, KRT10, a été stimulée (Tableau 1).

### Extrait culture cellule végétale (IBO.18.134)

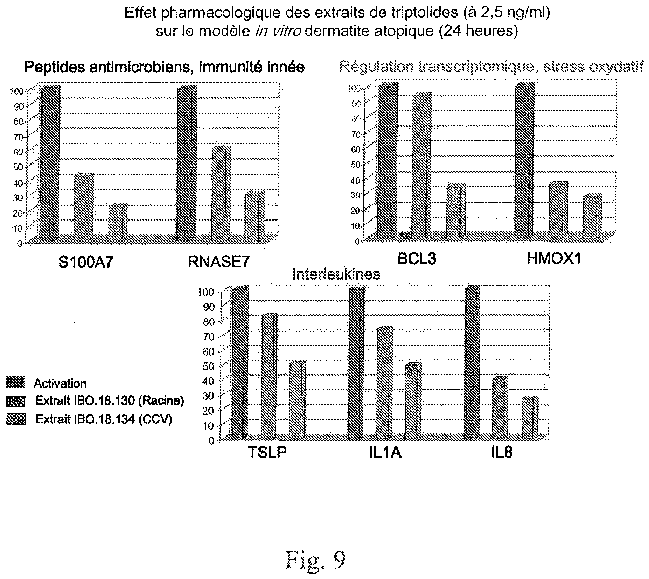

L'extrait de culture cellule végétale (CCV) présente un effet anti-inflammatoire plus marqué que l'extrait de racine en réprimant l'expression des marqueurs d'immunité innées S100A7 et RNASE7, des cytokines TSLP et IL1A, des chimiokines (CCL3, CCL5 et IL8) et du marqueur de stress oxidatif (HMOX1) (Figure 9).

**Tableau 1 : Gènes testés après induction d'un phénotype dermatite atopique**

| **Peptide anti-microbien, immunité innée** | |
|---|---|
| TLR3 | Toll-like receptor 3 |
| S100A7 | S100 calcium binding protein A7 |
| S100A11 | S100 calcium binding protein A11 |
| RNASE7 | Ribonuclease, RNase A family, 7 |
| CAMP | Cathelicidin antimicrobial peptide |

| **Interleukines** | |
|---|---|
| TSLP | Thymic stromal lymphopoietin |
| IL1A | Interleukin 1, alpha |
| IL18 | Interleukin 18 (interferon-gamma-inducing factor) |
| IFNA2 | Interferon, alpha 2 |
| IFNB1 | Interferon, beta 1, fibroblast |
| IL4R | Interleukin 4 receptor |

| **Chemokines** | |
|---|---|
| IL8 | Interleukin 8 |
| CCL3 | Chemokine (C-C motif) ligand 3 |
| CCL5 | Chemokine (C-C motif) ligand 5 |
| CCL7 | Chemokine (C-C motif) ligand 7 |
| CCL11 | Chemokine (C-C motif) ligand 11 |
| CCL13 | Chemokine (C-C motif) ligand 13 |
| CCL17 | Chemokine (C-C motif) ligand 17 |
| CCL20 | Chemokine (C-C motif) ligand 20 |
| CCL22 | Chemokine (C-C motif) ligand 22 |
| CCL27 | Chemokine (C-C motif) ligand 27 |

| **Keratinocyte differentiation** | |
|---|---|
| IVL | Involucrin |
| CDSN | Corneodesmosin |
| FLG | Filaggrin |
| LOR | Loricrin |
| KRT10 | Keratin 10 |
| LASS6 | LAG1 homolog, ceramide synthase 6 |

| **Régulation transcriptionnelle** | |
|---|---|
| RARRES3 | Retinoic acid receptor responder (tazarotene induced) 3 |
| BCL3 | B-cell CLL/lymphoma 3 |

| **Réponse au stress oxydatif** | |
|---|---|
| HMOX1 | Heme oxygenase (decycling) 1 |

### 2) Effets des composés sur des kératinocytes présentant un phénotype psoriatique

### 2.1 Validation de l'expérience

Le traitement des kératinocytes par le mélange de cytokines (Oncostatin M + IL17 + TNF) a clairement induit un phénotype de type psoriatique en induisant l'expression de différents gènes caractéristiques impliqués dans la pathologie.

Le mélange de cytokines induit une augmentation d'expression des gènes codant pour des peptides anti-microbiens ou impliqués dans l'immunité innée (CAMP, DEFB103A, DEFB4, PI3, S100A7, S100A7A, SPLI et TLR2), dans le chimiotactisme (CCL5, CXCL5, CXCL10 et IL8), dans l'inflammation (IL1B), la dégradation de la matrice extracellulaire (MMP1 et MMP3) et la défense cellulaire contre le stress oxydatif (HMOX1). Parallèlement une inhibition des marqueurs impliqués dans la différentiation (KRT1, KRT10 et FLG) et la cohésion cellulaire (DSG1 et CALML5) est observée.

### 2.2 Effet des composés sur le modèle Psoriasis

Les 2 composés ont été testés à la dose de 2,5 ng/ml (équivalent en concentration en triptolide pur).

### Extrait de racine (IB0.18.130)

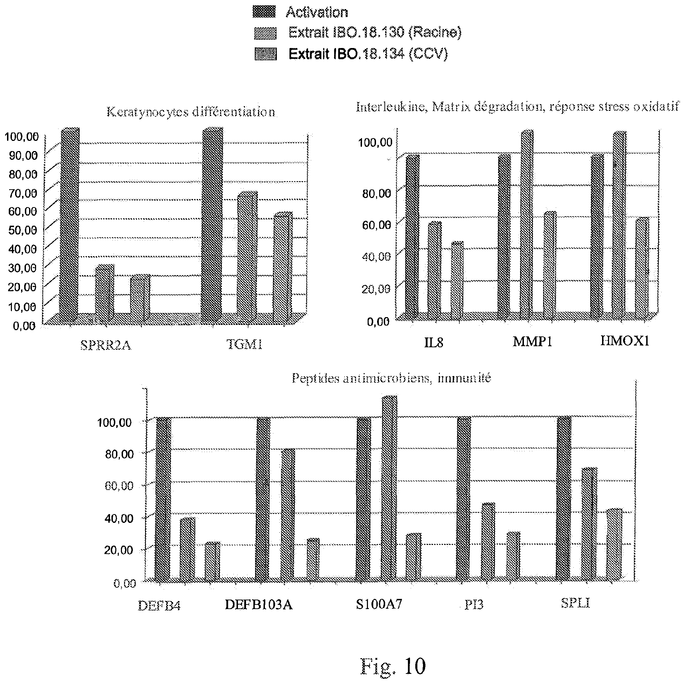

L'extrait de racine présente un effet anti-inflammatoire sur ce modèle. En effet, une dizaine de gènes induits par le cocktail de cytokines sont réprimés par l'extrait. Un effet dose sur certains gènes peut également être observé (Figure 10).

### Extrait culture cellule végétale (IBO.18.134)

L'extrait de culture cellule végétale (CCV) présente également un effet anti-inflammatoire sur ce modèle. En effet, treize gènes induits par le cocktail de cytokines sont réprimés d'un facteur >2 par l'extrait. L'effet anti-inflammatoire sur le modèle psoriasis est plus marqué avec l'extrait de culture végétale que l'extrait de racine (Figure 10).

**Tableau 2 : Gènes testés après induction d'un phénotype psoriasis**

| **Keratinocyte differentiation** | |
|---|---|
| CALML5 | Calmodulin-like 5 |
| FABP5 | Fatty acid binding protein 5 (psoriasis-associated) |
| FLG | Filaggrin |
| KRT1 | Keratin 1 |
| KRT10 | Keratin 10 |
| LOR | Loricrin |
| SPRR1A | Small proline-rich protein 1A |
| SPRR2A | Small proline-rich protein 2A |
| TGM1 | Transglutaminase 1 |

| **Peptide anti-microbien, immunité innée** | |
|---|---|
| CAMP | Cathelicidin antimicrobial peptide |
| DEFB103A | Defensin, beta 103A |
| DEFB4 | Defensin, beta 4 |
| PI3 | Peptidase inhibitor 3, skin-derived |
| RNASE7 | Ribonuclease, RNase A family, 7 |
| S100A7 | S100 calcium binding protein A7 |
| S100A7A | S100 calcium binding protein A7A |
| SLPI | Secretory leukocyte peptidase inhibitor |
| TLR2 | Toll-like receptor 2 |

| **Chemokines** | |
|---|---|
| CCL5 | Chemokine (C-C motif) ligand 5 |
| CXCL10 | Chemokine (C-X-C motif) ligand 10 |
| | Chemokine (C-X-C motif) ligand 5 |
| CXCL5 IL8 | Interleukin 8 |

| **Interleukines** | |
|---|---|
| IL1B | Interleukin 1, beta |

| **Cell-cell interactions** | |
|---|---|
| **CDSN** | Corneodesmosin |
| DSG1 | Desmoglein 1 |
| CEACAM1 | Carcinoembryonic antigen-related cell adhesion molecule 1 |

| **Réponse au stress oxydatif** | |
|---|---|
| HMOX1 | Heme oxygenase (decycling) 1 |

| **Matrix degradation/ Wound healing** | |
|---|---|
| MMP1 | Matrix metallopeptidase 1 (interstitial collagenase) |
| MMP3 | Matrix metallopeptidase 3 (stromelysin 1, progelatinase) |

| **STAT signalling** | |
|---|---|
| SOCS3 | Suppressor of cytokine signaling 3 |

### CONCLUSIONS SUR LES 2 MODELES

Dans les conditions expérimentales de cet essai, les extraits IBO.18.130 (extrait de racine) et IBO.18.134 (extrait culture végétale) ont présenté un effet anti-inflammatoire dans les modèles *in vitro* induits sur kératinocytes de dermatite atopique et de psoriasis. Cet effet est plus marqué avec l'extrait de culture végétale comparé à l'extrait de racine en équivalence de triptolide pur.

### C. TESTS DE CYTOTOXICITE

Comparaison de cytotoxicité de 3 composés : TRP (MP0001128), l'extrait de racine (IBO 18.130) et l'extrait CCV (IBO 18.134) à concentrations identiques en TRP.

Les cellules NHEK sont ensemencées dans des microplaques 96 puits, noire et incubées à 37°C, 5% CO2 pendant 24h.

Les microplaques 96 puits sont centrifugées 10 minutes à 1200 RPM

### Dosage ATPlite

50µl de tampon de lyse sont ajoutés dans la microplaque noire, après agitation 50µl de substrat sont ajoutés et la luminescence est lue au TopCount.

### Dosage LDH

100µl de surnageant sont prélevés de la microplaque noire et déposés dans une microplaque transparente. Après ajout de 100µl de la solution de dosage LDH la plaque est incubée 30 minutes à TA, à l'abri de la lumière. La réaction colorimétrique du dosage LDH est arrêtée avec 50µl de HCl 1N. L'analyse de la cytolyse est donnée par lecture de l'absorbance sur le lecteur Envision à 485nm.

Les cellules NHEK sont mises en contact avec des doses croissantes des différents composés.

Les résultats obtenus par le test LDH et celui de l'ATPlite montrent clairement une cytotoxicité plus importante du IBO.18.130 (extrait de racine) par rapport au IBO.18.134 (extrait de culture végétale) et au MP00011128 (Triptolide pur).

## Revendications

1. Procédé de production de Triptolide dans le milieu de culture à partir d'une culture cellulaire de l'espèce *Tripterygium* comprenant les étapes suivantes :
(i) une phase de production de biomasse de cellules dédifférenciées de l'espèce *Tripterygium* dérivées de cals dans un ou des milieux nutritifs dans des conditions de multiplication de la biomasse,
(ii) une phase de sevrage hormonal des cultures cellulaires obtenues à l'étape (i) dans un milieu de sevrage dans lequel l'acide 2,4 dichlorophénoxyacétique (2,4D) et l'acide naphthalène acétique (NAA) sont présents respectivement à une concentration inférieure à 0,01 mg/L de milieu de culture,
(iii) une phase d'élicitation par ajoût d'un cocktail d'élicitation aux cellules de l'étape (ii) dans du milieu de sevrage, le cocktail d'élicitation comprenant :
a) au moins un facteur de différenciation cellulaire des cellules végétales,
b) au moins un agent stressant, et
c) au moins un précurseur de la voie de synthèse des terpènes,
(iv) préparation d'un extrait enrichi en triptolide à partir du milieu de culture à l'issue de l'étape (iii).

2. Procédé selon la revendication 1, **caractérisé en ce que** le milieu de sevrage ne contient pas d'auxines.

3. Procédé selon la revendication 2, **caractérisé en ce que** le facteur de différenciation cellulaire de cellule végétale est choisi parmi les cytokinines.

4. Procédé selon la revendication 2 ou 3, **caractérisé en ce que** l'agent stressant est un éliciteur abiotique.

5. Procédé selon la revendication 4, **caractérisé en ce que** l'éliciteur abiotique comprend de l'acide 5-chloro salycilique, de l'acide acétyl salicylique ou de l'acide salicylique et du méthyl-jasmonate.

6. Procédé selon l'une quelconque des revendications 2 à 5, **caractérisé en ce que** le précurseur de la voie de synthèse des terpènes est choisi dans le groupe comprenant le farnesol, le géraniol, l'acétate de sodium, l'acide pyruvique ou l'acide mévalonique.

7. Procédé selon l'une quelconque des revendications 1 à 6, **caractérisé en ce que** le milieu de sevrage ne contient ni d'acide 2,4 dichlorophénoxyacétique (2,4D), ni d'acide Naphthalène acétique (NAA).

8. Procédé selon l'une quelconque des revendications 1 à 7, **caractérisé en ce que** l'étape de sevrage hormonal (ii) dure entre 5 et 15 jours, par exemple 7 jours.

9. Procédé selon l'une quelconque des revendications 1 à 8, **caractérisé en ce que** la phase d'élicitation (iii) dure de 15 à 35 jours, par exemple 20 à 25 jours.

10. Procédé de production de triptolide selon l'une quelconque des revendications précédentes, **caractérisé en ce que** la phase de production de la biomasse (i) se déroule sous agitation pendant 10-30 jours à environ 27-28°C.

11. Procédé de production de triptolide selon l'une quelconque des revendications précédentes, **caractérisé en ce que** le cocktail d'élicitation contient de la benzyl-aminopurine, de l'acide 5-chloro salicylique, de l'acide acetyl-salicylique, ou de l'acide salicylique, du méthyl-jasmonate, du farnesol et du géraniol.

12. Procédé de production de triptolide selon l'une quelconque des revendications précédentes, **caractérisé en ce que** l'étape (iv) est une extraction liquide/liquide.

13. Utilisation d'un cocktail d'élicitation comprenant :
a) au moins un facteur de différenciation cellulaire des cellules végétales,
b) au moins un agent stressant, et
c) au moins un précurseur de la voie de synthèse des terpènes,
pour la culture cellulaire de l'espèce *Tripterygium.*

14. Utilisation selon la revendication 13, ledit cocktail d'élicitation comprenant : Benzyl-aminopurine (BAP) de 0,7 à 3 mg/L, Acide 5-Chloro Salicylique (5-Chloro SA) de 3 à 5 mg/L, Acide Acetyl-Salicylique (ASA) de 33 à 45 mg/L, Methyl-Jasmonate (MeJA) de 22,4 à 24 mg/L, Géraniol de 20 à 30 mg/L, et Farnesol (F-OH) de 19 à 30 mg/L, avec mg/L exprimant des mg/L de milieu de culture.

15. Extrait enrichi en triptolide susceptible d'être obtenu par extraction du milieu de culture d'une culture *in vitro* de cellules dédifférenciées de l'espèce *Tripterygium* par le procédé selon la revendication 12, la teneur en triptolide du surnageant de culture après l'étape (iii) du procédé étant supérieure à 50 mg/litre.

## Patentansprüche

1. Verfahren für die Herstellung von Triptolid in Kulturmedium auf der Basis einer Zellkultur der Spezies *Tripterygium,* umfassend die folgenden Schritte:
(i) eine Biomasse-Produktionsphase dedifferenzierter Zellen der Spezies *Tripterygium* als Kallusderivat in einem oder in Nährmedien unter Biomasse-Vermehrungsbedingungen,
(ii) eine hormonale Entzugsphase der in Schritt (i) gewonnenen Zellkulturen in einem Entzugsmedium, in welchem die 2,4-Dichlorphenoxyessigsäure (2,4D) und die Naphthalenessigsäure (NAA) jeweils in einer Konzentration unter 0,01 mg/L Kulturmedium vorhanden sind,
(iii) eine Elicitationsphase durch Hinzufügen eines Elicitationscocktails zu den Zellen von Schritt (ii) in das Entzugsmedium, wobei der Elicitationscocktail umfasst:
a) mindestens einen Zelldifferenzierungsfaktor der Pflanzenzellen,
b) mindestens ein Stressmittel, und
c) mindestens einen Vorläufer des Terpen-Synthesewegs,
(iv) Zubereitung eines mit Triptolid angereicherten Extrakts auf der Basis des Kulturmediums nach Abschluss von Schritt (iii).

2. Verfahren nach Anspruch 1, **dadurch gekennzeichnet, dass** das Entzugsmedium keine Auxine enthält.

3. Verfahren nach Anspruch 2, **dadurch gekennzeichnet, dass** der Zelldifferenzierungsfaktor der Pflanzenzelle aus den Cytokininen ausgewählt ist.

4. Verfahren nach Anspruch 2 oder 3, **dadurch gekennzeichnet, dass** das Stressmittel ein abiotischer Elicitor ist.

5. Verfahren nach Anspruch 4, **dadurch gekennzeichnet, dass** der abiotische Elicitor 5-Chlorsalycylsäure, Acetylsalicylsäure oder Salicylsäure und Methyljasmonat umfasst.

6. Verfahren nach einem der Ansprüche 2 bis 5, **dadurch gekennzeichnet, dass** der Vorläufer des Terpen-Synthesewegs aus der Gruppe ausgewählt ist, die das Farnesol, das Geraniol, das Natriumacetat, die Pyruvinsäure oder die Mevalonsäure umfasst.

7. Verfahren nach einem der Ansprüche 1 bis 6, **dadurch gekennzeichnet, dass** das Entzugsmedium weder 2,4-Dichlorphenoxyessigsäure (2,4D) noch Naphthalenessigsäure (NAA) enthält.

8. Verfahren nach einem der Ansprüche 1 bis 7, **dadurch gekennzeichnet, dass** der hormonale Entzugsschritt (ii) 5 bis 15 Tage, beispielsweise 7 Tage, dauert.

9. Verfahren nach einem der Ansprüche 1 bis 8, **dadurch gekennzeichnet, dass** die Elicitationsphase (iii) 15 bis 35 Tage, beispielsweise 20 bis 25 Tage, dauert.

10. Verfahren für die Herstellung von Triptolid nach einem der vorangehenden Ansprüche, **dadurch gekennzeichnet, dass** die Biomasse-Produktionsphase (i) unter Rühren während 10-30 Tagen bei zirka 27-28 °C erfolgt.

11. Verfahren für die Herstellung von Triptolid nach einem der vorangehenden Ansprüche, **dadurch gekennzeichnet, dass** der Elicitationscocktail Benzylaminopurin, 5-Chlorsalicylsäure, Acetylsalicylsäure oder Salicylsäure, Methyljasmonat, Farnesol und Geraniol enthält.

12. Verfahren für die Herstellung von Triptolid nach einem der vorangehenden Ansprüche, **dadurch gekennzeichnet, dass** der Schritt (iv) eine Flüssigkeit/Flüssigkeit-Extraktion ist.

13. Verwendung eines Elicitationscocktails, umfassend:
a) mindestens einen Zelldifferenzierungsfaktor der Pflanzenzellen,
b) mindestens ein Stressmittel, und
c) mindestens einen Vorläufer des Terpen-Synthesewegs,
für die Zellkultur der Spezies *Tripterygium.*

14. Verwendung nach Anspruch 13, wobei der Elicitationscocktail umfasst: Benzylaminopurin (BAP) von 0,7 bis 3 mg/L, 5-Chlorsalicylsäure (5-Chlor SA) von 3 bis 5 mg/L, Acetylsalicylsäure (ASA) von 33 bis 45 mg/L, Methyljasmonat (MeJA) von 22,4 bis 24 mg/L, Geraniol von 20 bis 30 mg/L und Farnesol (F-OH) von 19 bis 30 mg/L, wobei mg/L mg/L Kulturmedium ausdrückt.

15. Extrakt, angereichert mit Triptolid, herstellbar durch Extraktion des Kulturmediums einer *in vitro*-Kultur dedifferenzierter Zellen der Spezies *Tripterygium* mittels des Verfahrens nach Anspruch 12, wobei der Triptolidgehalt des Kulturüberstands nach Schritt (iii) über 50 mg/Liter beträgt.

## Claims

1. A method for producing triptolide in culture medium from a cell culture of the species *Tripterygium* comprising the following steps:
(i) a phase of production of a biomass of dedifferentiated cells of the species *Tripterygium* derived from calluses in nutrient medium or media under biomass growth conditions,
(ii) a phase of hormonal elimination of the cell cultures obtained in step (i) in an elimination medium wherein 2,4-dichlorophenoxyacetic acid (2,4-D) and naphthalene acetic acid (NAA) are present respectively at a concentration lower than 0.01 mg/L of culture medium,
(iii) a phase of elicitation by addition of an elicitation cocktail to the cells of step (ii) in elimination medium, the elicitation cocktail comprising :
a) at least one cellular differentiation factor of plant cells,
b) at least one stressing agent, and
c) at least one precursor of the terpene synthesis pathway,
(iv) preparation of a triptolide-enriched extract from the culture medium at the end of step (iii).

2. The method of claim 1, wherein the elimination medium contains no auxin.

3. The method of claim 2, wherein the cellular differentiation factor of plant cells is selected from the cytokinins.

4. The method of claim 2 or 3, wherein the stressing agent is an abiotic elicitor.

5. The method of claim 4, wherein the abiotic elicitor comprises 5-chlorosalicylic acid, acetylsalicylic acid or salicylic acid, and methyl jasmonate.

6. The method of any one of claims 2 to 5, wherein the precursor of the terpene synthesis pathway is selected from the group comprising farnesol, geraniol, sodium acetate, pyruvic acid or mevalonic acid.

7. The method of any one of claims 1 to 6, wherein the elimination medium contains no 2,4-dichlorophenoxyacetic acid (2,4-D) or naphthalene acetic acid (NAA).

8. The method of any one of claims 1 to 7, wherein the hormonal elimination step (ii) lasts for 5-15 days, for example 7 days.

9. The method of any one of claims 1 to 8, wherein the elicitation phase (iii) lasts for 15-35 days, for example 20-25 days.

10. The method of triptolide production of any one of the preceding claims, wherein the biomass production phase (i) proceeds with agitation for 10-30 days at roughly 27-28°C.

11. The method of triptolide production of any one of the preceding claims, wherein the elicitation cocktail contains benzylaminopurine, 5-chlorosalicylic acid, acetylsalicylic acid or salicylic acid, methyl jasmonate, farnesol and geraniol.

12. The method of triptolide production of any one of the preceding claims, wherein the step (iv) is liquid/liquid extraction.

13. The use of an elicitation cocktail comprising:
a) at least one cellular differentiation factor of plant cells,
b) at least one stressing agent, and
c) at least one precursor of the terpene synthesis pathway,
to culture of cells of the species *Tripterygium.*

14. The use according to claim 13, wherein said elicitation cocktail comprises: 0.7-3 mg/L benzylaminopurine (BAP); 3-5 mg/L 5-chlorosalicylic acid (5-chloro SA); 33-45 mg/L acetylsalicylic acid (ASA); 22.4-24 mg/L methyl jasmonate (MeJA); 20-30 mg/L geraniol; and 19-30 mg/L farnesol (F-OH); with mg/L expressing mg/L of culture medium.

15. A triptolide-enriched extract obtainable by extraction from the culture medium of an *in vitro* culture of dedifferentiated cells of the species *Tripterygium* by the method of claim 12, wherein the triptolide concentration in the culture supernatant after step (iii) is greater than 50 mg/liter.
